# EUROPÄISCHE PATENTSCHRIFT

(11) **EP 0 543 165 B1**
(45) Veröffentlichungstag und Bekanntmachung des Hinweises auf die Patenterteilung: **30.11.1994**
(21) Anmeldenummer: 92118014.7
(22) Anmeldetag: 21.10.1992
(51) Int. Cl.: A61B 5/11, A61B 5/0428

(54) **Vorrichtung zum Messen der Herzwandbewegungen einer Person**
Device for monitoring the movements of cardiac wall segments of a person
Dispositif de surveillance de mouvements de la paroi cardiaque d'un sujet

(30) Priorität: 18.11.1991 DE 4137951; 28.08.1992 DE 4228766
(43) Veröffentlichungstag der Anmeldung: 26.05.1993
(73) Patentinhaber: LRE RELAIS + ELEKTRONIK GMBH, D-80335 München (DE)
(72) Erfinder: Deuter, Klaus, Dr., W-8050 Freising (DE); Körner, Jochen, W-8000 München 50 (DE)
(74) Vertreter: Schaumburg, Thoenes & Thurn

(56) Entgegenhaltungen:
- EP-A- 0 178 197
- US-A- 3 882 846
- US-A- 3 945 373
- US-A- 4 182 315
- AEROSPACE MEDICINE Juli 1968, Seiten 745 - 750 P.C.RICHARDSON ET AL. 'Some New Electrode Techniques for Long-term Physiologic Monitoring' S. 749-759, Abschnitt "Insulated Electrodes"

## Beschreibung

Die Erfindung betrifft eine Vorrichtung zum Messen der Herzwandbewegungen einer Person, umfassend einen an die Brust anlegbaren Meßkopf mit einer Elektrodenanordnung und einer Meßschaltung zur kapazitiven Messung der Hautbewegungen gegenüber der Elektrodenanordnung.

Eine Vorrichtung der vorstehend genannten Art, wie sie z.B. aus der Druckschrift US-A- 4 182 315 bekannt ist, dient zur Erstellung eines sogenannten Cardiokymogrammes (CKG), das insbesondere in Verbindung mit einem Elektrocardiogramm (EKG) Auskunft darüber geben kann, ob sich der Herzmuskel auch bei Anregung durch die im EKG sichtbaren Impulse ordnungsgemäß kontrahiert. Die Herzwandbewegung setzt sich im Gewebe fort und läßt sich an der Brustoberfläche als eine sehr geringfügige Bewegung der Haut feststellen. Diese Bewegung kann beispielsweise dadurch erfaßt werden, daß die Hautoberfläche zusammen mit einer Elektrode einen Kondensator bildet, der im frequenzbestimmenden Teil eines Oszillators liegt. Eine Bewegung der Haut verursacht eine Änderung der Kapazität dieses Kondensators und damit eine Änderung der Schwingungsfrequenz des Oszillators. Die dadurch hervorgerufene Frequenzmodulation läßt sich zur Gewinnung eines die Herzkammerwandbewegung darstellenden Signales auswerten.

Die zu erfassende Bewegung hat eine sehr geringe Amplitude und wird daher nicht nur durch die Eigenschaften des Gewebes und der Haut beeinflußt, sondern auch durch die von der Atmung hervorgerufenen oder sonstigen Körperbewegungen überdeckt. Mit einer bisher bekannten Vorrichtung der eingangs genannten Art war es nur unter Laborbedingungen durch absolute Ruhigstellung des zu untersuchenden Patienten möglich, brauchbare Signale zu erhalten. Damit war es aber bisher nicht möglich, ein CKG bei gleichzeitiger Körperbelastung des Patienten zu erstellen. Häufig können aber Herzfehler gerade erst bei körperlicher Belastung des Patienten entdeckt werden. Dazu sind bereits tragbare Meßeinrichtungen geschaffen, welche die Erstellung eines 24-Stunden-EKGs ermöglichen. Die parallele Aufnahme eines CKG über den gleichen Zeitraum war mit der bisher bekannten Meßvorrichtung nicht möglich.

Der Erfindung liegt die Aufgabe zugrunde, eine Vorrichtung der eingangs genannten Art anzugeben, die am Körper der zu untersuchenden Person getragen werden kann und weitgehend unempfindlich gegen Störeinflüsse ist, die durch die Atmung und die sonstigen Bewegungen der Person verursacht werden.

Diese Aufgabe wird erfindungsgemäß dadurch gelöst, daß der Meßkopf einen Halterungsring umfaßt, der eine zur Anlage am Körper bestimmte, zur Ringachse senkrechte Ringfläche und Mittel zur Befestigung des Meßkopfes am Körper hat, daß in dem Halterungsring ein die Elektrodenanordnung umfassender Adapter in Richtung der Ringachse sowie um zwei zu dieser und zueinander senkrechte Achsen federn beweglich gelagert ist und daß der Adapter eine zur Anlage am Körper bestimmte ringförmige Auflagefläche und einen von dieser umschlossenen Elektrodenträger mit einer mindestens eine Elektrode tragenden Elektrodenträgerfläche hat, die senkrecht zur Ebene der Auflagefläche einen Abstand hat und die mit einem Abstandselement versehen ist.

Mit Hilfe des Halterungsringes kann der Meßkopf unverrückbar am Körper der Person befestigt werden. Die Befestigung kann beispielsweise durch am Halterungsring angreifende Brust- und Schultergurte erfolgen. Da der Adapter gegenüber dem Halterungsring federnd beweglich ist und an dem Halterungsring quasi kardanisch gelagert ist, kann er sich zum einen der Körperform im Meßbereich in seiner Lage anpassen und wird zum anderen unabhängig von der Spannung, mit welcher der Halterungsring am Körper anliegt, allein durch die vorgegebene Federkraft an den Körper angedrückt. Dies ermöglicht es, einerseits den Meßkopf am Körper so festzulegen, daß er durch die üblichen Körperbewegungen nicht verrutschen kann, und andererseits einen Abstand zwischen der Haut und der Elektrode einzuhalten, der ein Meßsignal in dem gewünschten Bereich liefert. Dabei gewährleistet das Abstandselement einen relativ großflächigen Meßbereich unter Einhaltung eines annähernd konstanten Abstandes zwischen der Haut und der Elektrodenoberfläche innerhalb der Meßfläche.

Da die Festigkeitseigenschaften der Haut und des darunterliegenden Gewebes bei den zu untersuchenden Personen sehr unterschiedlich sein kann, ist es zweckmäßig, wenn der Abstand zwischen der Elektrode und der Haut nach dem Anlegen des Meßkopfes individuell eingestellt werden kann. Hierzu ist vorgesehen, daß der Elektrodenträger relativ zur Auflagefläche in Richtung der Ringachse, d.h. im wesentlichen senkrecht zur Körperoberfläche verstellbar ist. Vorzugsweise hat dabei der Adapter ein topfförmiges Gehäuse, an dessen Öffnungsrand die Auflagefläche ausgebildet ist, während innerhalb des Gehäuses der Elektrodenträger zusammen mit der Meßschaltung angeordnet ist. In diesem Fall kann die Verstellung auf einfache Weise dadurch erfolgen, daß der Elektrodenträger durch Federmittel in Richtung auf den Topf- oder Gehäuseboden vorgespannt und durch eine Stellvorrichtung von dem Gehäuseboden weg bewegbar ist. Bei einer einfach herzustellenden und zu bedienenden Ausführungsform ist der Gehäuseboden gegenüber der Gehäusewand um die Ringachse drehbar und trägt an seiner Innenseite mindestens einen Nocken, der mit einer gegenüber einer achsnormalen Ebene geneigten Steuerfläche an dem Elektrodenträger zusammenwirkt. Durch eine Drehung des Gehäusebodens wird damit durch die geneigte, beispielsweise rampenförmige Steuerfläche an dem Elektrodenträger dieser in axialer Richtung verstellt.

Der Anschluß der die zweite Kondensatorelektrode bildenden Haut der Person an die Meßschaltung erfolgt im einfachsten Falle dadurch, daß die ringförmige Auflagefläche des Adapters elektrisch leitend ist, indem beispielsweise die Gehäusewand des Adaptergehäuses aus Metall hergestellt wird.

Eine wesentliche Steigerung der Funktionssicherheit des erfindungsgemäßen Meßkopfes läßt sich dadurch erreichen, daß zwei Elektroden vorgesehen sind, die beispielsweise ringförmig ausgebildet und konzentrisch zueinander auf der Trägerfläche um die zentrale Erhebung herum angeordnet sind, wobei jede dieser Elektroden einem Oszillator der Meßanordnung zugeordnet ist. Die Auswertung der Meßsignale kann dabei so erfolgen, daß jeder Oszillator jeweils die Hälfte des Signales liefert. Wenn eine der Elektroden kurzgeschlossen werden sollte, weil die Haut die Elektrode berührt und somit der betreffende Oszillator kein Meßsignal mehr liefert, ermöglicht dann zumindest der andere Oszillator die Fortführung der Messung.

Alternativ hierzu können beide Elektroden auch an einen Oszillator derart angeschlossen sein, daß jede Einzelkapazität die Dauer einer Halbwelle des Ausgangssignales bestimmt. Auch bei dieser Ausführungsform kann die Messung bei an einer Elektrode anliegender Haut weitergeführt werden.

Weitere Merkmale und Vorteile der Erfindung ergeben sich aus der folgenden Beschreibung, welche in Verbindung mit den beigefügten Figuren die Erfindung anhand von Ausführungsbeispielen erläutert. Es zeigen:
- Figur 1: eine vereinfachte perspektivische Darstellung des Meßkopfes,
- Figur 2: eine Draufsicht auf die bei der Messung der Person zugewandte Vorderseite des Meßkopfes,
- Figur 3: einen die Achse enthaltenden Schnitt durch den erfindungsgemäßen Meßkopf entlang Linie III-III in Figur 2, wobei der Meßkopf in seiner am Körper anliegenden Stellung dargestellt ist,
- Figur 4: eine Draufsicht auf eine Feder zwischen dem Adapter und dem Halterungsring,
- Figur 5: eine Seitenansicht der in Figur 4 dargestellten Feder,
- Figur 6: eine schematische Darstellung des Meßprinzips,
- Figur 7: ein weiteres Ausführungsbeispiel mit ebener Elektrodenträgerfläche und
- Figur 8: eine Draufsicht auf die elastische Aufhängung beim Ausführungsbeispiel nach Figur 7.

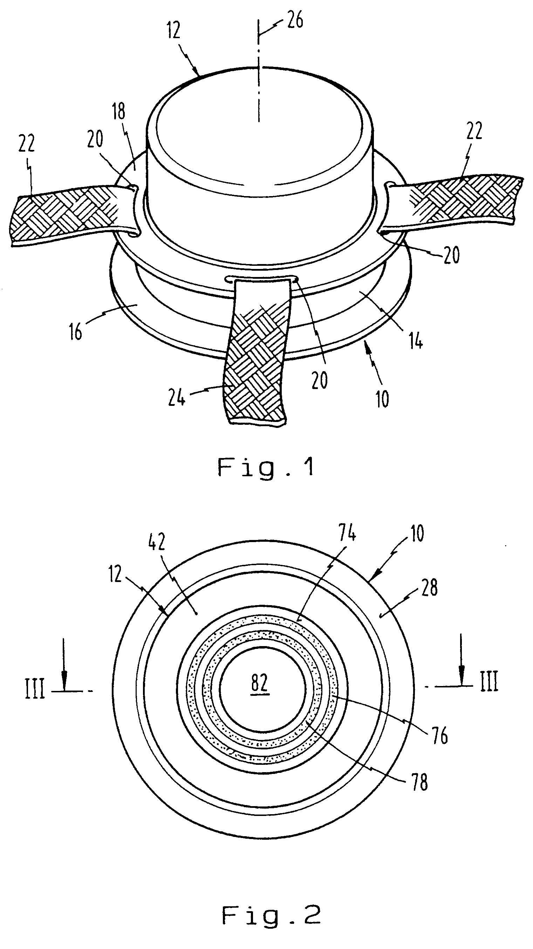

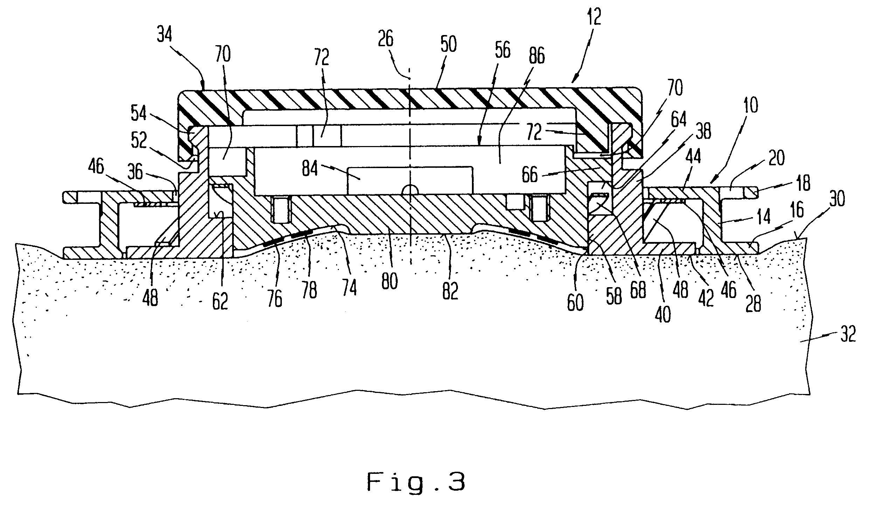

Der in der Figur 1 dargestellte Meßkopf umfaßt einen allgemein mit 10 bezeichneten Halterungsring und einen in diesem in begrenztem Umfange beweglich gelagerten Adapter 12, der die eigentliche Meßvorrichtung enthält. Der Halterungsring 10 umfaßt eine zylindrische Wand 14 mit einem ersten radial auswärts gerichteten Flansch 16 und einem zweiten radial auswärts gerichteten Flansch 18. In letzterem sind Schlitze 20 ausgebildet, an denen in der Figur 1 nur abschnittsweise dargestellte Teile eines Brustgurtes 22 und eines Schultergurtes 24 befestigt sind, mit denen der Meßkopf an der Brust eines Patienten unverückbar festgelegt werden kann. Dabei liegt der Halterungsring 10 mit einer senkrecht zu seiner Achse 26 gerichteten Ringfläche 28 an der Haut 30 (Figur 3) der Person an und wird durch den Zug der Gurte 22, 24 je nach Festigkeit des Gewebes 32 mehr oder weniger tief in dieses eingedrückt, wie das die Figur 3 zeigt.

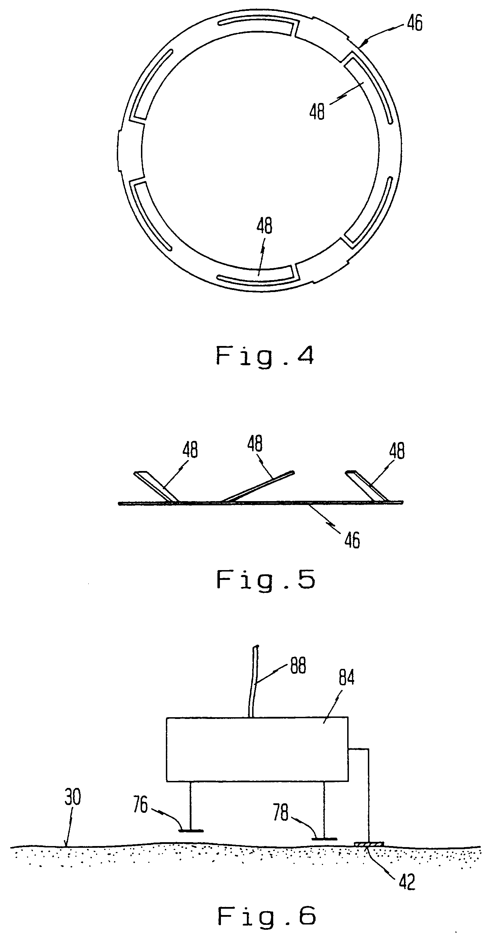

Der Adapter 12 umfaßt ein topfförmiges Gehäuse 34 und ist innerhalb der kreisförmigen Öffnung 36 des Halterungsringes 10 angeordnet. Das Gehäuse 34 besteht im vorliegenden Ausführungsbeispiel aus einer im wesentlichen zylindrischen Wand 38 mit einem radial auswärts gerichteten Flansch 40, an dem eine dem Patienten zugewandte ringförmige Auflagefläche 42 ausgebildet ist. Der Flansch 40 der Gehäusewand 38 überdeckt in radialer Richtung einen einwärts gerichteten Ringflansch 44 des Halterungsringes 10. Zwischen den beiden Flanschen 44 und 40 liegt ein Federring 46, der in den Figuren 4 und 5 dargestellt ist. Er besteht aus einem Federblechring, aus dem in Umfangsrichtung verlaufende Federzungen 48 ausgestanzt und gemäß Figur 5 aus der Ringebene schräg herausgebogen sind. Im vorliegenden Beispiel sind es sechs derartige Federzungen, auf denen der Flansch 44 des Adaptergehäuses 34 aufliegt. Wie man erkennt, kann sich dadurch der Adapter 12 relativ zum Halterungsring 10 sowohl in Richtung der Achse 26 bewegen als auch um diese Achse 26 taumeln, so daß er unter der durch den Federring 46 bestimmten Vorspannung an der Haut anliegt und sich dabei der Form des innerhalb des Halterungsringes 10 liegenden Körperabschnittes anpassen kann. Man erkennt ferner, daß der Druck, mit dem der Adapter 12 an die Hautoberfläche 30 angepreßt wird, praktisch unabhängig davon ist, mit welchem Druck der Halterungsring 10 mittels der Gurte 22 und 24 an den Körper angepreßt wird.

Das Adaptergehäuse 34 umfaßt ferner einen Deckel 50 der beispielsweise aus Kunststoff hergestellt sein kann und mittels einer radial einwärts gerichteten Ringrippe 52 über eine korrespondierende radial auswärts gerichtete Ringrippe 54 an der Gehäusewand 38 aufschnappen kann. Der Deckel 50 ist damit zwar axial an der Gehäusewand 38 gehalten, kann jedoch gegenüber dieser um die Achse 26 verdreht werden.

Innerhalb des topfförmiges Gehäuses 34 ist ein allgemein mit 56 bezeichneter Elektrodenträger gehalten und mit einer zylindrischen Außenwand 58 an einer zylindrischen Innenwand 60 der Gehäusewand 38 geführt.

Zwischen einer zum Gehäuseboden 50 hinweisenden Stufenfläche 62 der Gehäusewand 38 und einer ihr gegenüberliegenden Stützfläche 64 eines auswärts gerichteten Flansches 66 des Elektrodenträgers 56 ist eine Ringfeder 68 angeordnet, welche den Elektrodenträger 56 in Richtung auf den Deckel 50 spannt und eine elektrisch leitende Verbindung zwischen der Auflagefläche 42 und der Meßschaltung 84 herstellt.

An der dem Deckel 50 zugewandten Rückseite des Elektrodenträgers 56 sind in Kreisrichtung verlaufende Rampenflächen 70 ausgebildet, auf denen Nocken 72 an der Innenseite des Deckels 50 gleiten. Durch eine Drehung des Deckels 50 drücken die Nocken 72 in Verbindung mit den Rampenflächen 70 den Elektrodenträger 56 gegen die Vorspannung der Ringfeder 68 mehr oder weniger weit nach vorne, wodurch der Elektrodenträger 56 relativ zum Adaptergehäuse 34 in axialer Richtung verstellt werden kann.

Auf der dem Körper des Patentien zugewandten Vorderseite ist an dem Elektrodenträger 56 eine konkav einwärts gewölbte Trägerfläche 74 ausgebildet, auf der zwei ringförmige, vorzugsweise flächengleiche Elektroden 76, 78 konzentrisch zur Achse 26 angeordnet sind. Im Zentrum der Trägerfläche 74 befindet sich eine Erhebung 80 mit einer achsnormalen Endfläche 82. Diese Erhebung 80 wirkt als Abstandshalter, der dafür sorgt, daß das sich in das Adaptergehäuse 34 hineinwölbende Gewebe 32 in einem Abstand von der Trägerfläche 74 gehalten wird, so daß die Haut 30 die Elektroden 76 und 78 nicht berührt. Der axiale Abstand der Endfläche 82 von der Trägerfläche 74, d.h. die relative Höhe der Erhebung 80 über der Trägerfläche 74 hängt von dem Gesamtdurchmesser der Trägerfläche und dem Gesamtdurchmesser der Erhebung 80 ab. Bei einem Gesamtdurchmesser der Trägerfläche 74 von ca. 45 mm und einem Durchmesser der Erhebung von 15 mm hat es sich als zweckmäßig herausgestellt, wenn die axiale Höhe der Erhebung zwischen ca. 1,3 bis 2,2 mm, vorzugsweise 1,7 mm beträgt.

Die Elektroden 76 und 78 sind mit einer Meßschaltung 84 verbunden, die in einer rückwärtigen Aussparung 86 in dem Elektrodenträger 65 angeordnet ist. Die Elektroden 76 und 78 bilden mit der Haut 30 jeweils einen Kondensator, der Teil eines Oszillators ist. Die elektrische Verbindung der Meßschaltung 84 und der Haut 30 erfolgt über die metallische Gehäusewand 38 und die Ringfeder 68, wie dies in Figur 6 schematisch dargestellt ist. Bei einer Bewegung der Haut, wie sie durch eine Herzwandbewegung hervorgerufen werden kann, wird die Kapazität der Kondensatoren verändert, so daß sich die Frequenzen der Oszillatoren ebenfalls ändern. Aus der daraus resultierenden Frequenzmodulation der Oszillatorfrequenzen läßt sich ein Meßsignal ableiten. Die Meßsignale können von der Meßschaltung 84 über eine aus dem Meßkopf herausgeführte Leitung 88 an eine nicht dargestellte Aufzeichnungseinheit übermittelt werden.

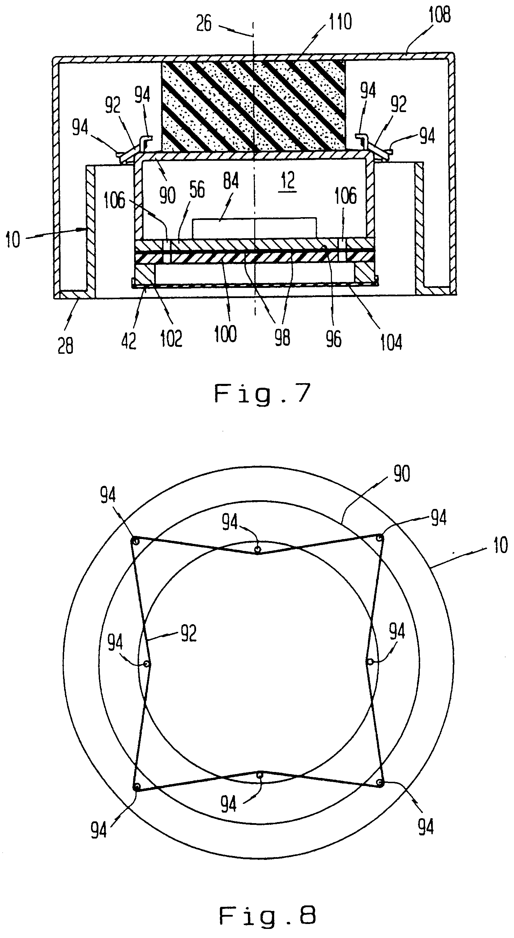

In Figur 7 ist ein weiteres Ausführungsbeispiel der Erfindung dargestellt, bei dem gleiche Teile gleich bezeichnet sind. Bei diesem Ausführungsbeispiel ist der Adapter 12 mit seinem Adaptergehäuse 90 im Halterungsring 10 durch ein Gummiband 92 federnd gelagert, das an Haken 94 am Adapter 12 und am Halterungsring 10 an mehreren Stellen abwechselnd gehalten ist. Die Haken 94 sind längs des Umfangs des Adaptergehäuses 90 bzw. des Halterungsrings 10 in gleichen Abständen gegeneinander versetzt angeordnet, so daß das Gummiband 92 um die Haken 94 geschlungen werden kann. Bei dieser Art der federnden Lagerung ist der Adapter 12 vollkardanisch im Halterungsring 10 aufgehängt.

Der Elektrodenträger 56 des Adapters 12 hat eine ebene Elektrodenträgerfläche 96, die mit zwei halbkreisförmigen Elektroden 98 versehen ist. Als Abstandselement ist eine Scheibe 100 aus Kunststoff vorgesehen, die einen Grundabstand von ca. 0,5 mm zwischen Haut und den Elektroden 98 herstellt, wenn die Haut einer zu untersuchenden Person gegen die Scheibe 100 drückt. Dadurch ist gewährleistet, daß auch bei einem teilweise Anliegen der Haut an der Scheibe 100 die Hautbewegung als Kapazitätsänderung durch die Elektroden 98 registriert wird.

Der senkrechte Abstand zwischen der Auflagefläche 42 und der Elektrodenträgerfläche 96 wird ferner durch ein Ringelement 102 bestimmt, das am Rand der Elektrodenträgerfläche 96 angeordnet ist. Zur Anpassung an verschiedene Haut- und Gewebeeigenschaften der zu untersuchenden Personen kann das Ringelement verschiedene Höhen haben, beispielsweise 1,5, 2,5 oder 3,5 mm, und als Austauschteil ausgebildet sein. Die zum Körper zeigende Öffnung des Ringelements 102 ist mit einer elastischen Schutzkappe 104 verhüllt, die beispielsweise aus einem gummiähnlichen Kunststoff besteht. Diese Schutzkappe 104 erfüllt hygienische Zwecke und kann leicht gegen eine andere ausgetauscht werden. Ferner verbessert diese Schutzkappe die Langzeitstabilität des Abstands zwischen Haut und Elektrodenträgerfläche 96.

Der zwischen der Schutzkappe 104, dem Ringelement 102 und der Scheibe 100 gebildete Raum ist über zwei Entlüftungsöffnungen 106 mit dem Außenraum verbunden, so daß dieser Raum bei Änderung seines Volumens infolge einer Einbeulung der Schutzkappe 104 belüftet und wieder entlüftet werden kann.

Der Halterungsring 10 trägt eine Gehäusekappe 108. Zwischen dieser Gehäusekappe 108 und dem Adaptergehäuse 90 ist eine Schaumgummieinlage 110 angeordnet, die Schwingungen des Adapters 12 dämpft. Die Gehäusekappe 108 ist so geformt, daß sie mittels eines breiten Gummibands (nicht dargestellt), das über die Gehäusekappe 108 geführt ist, den Halterungsring 10 gegen die Haut drückt. Das Gummiband ist vorzugsweise ein Endlosband, das den Körper oder Person in Brusthöhe umfaßt.

Auf der von dem Körper der zu untersuchenden Person abgewandten Seite des Elektrodenträgers 56 ist die Meßschaltung 84 angeordnet. Vorzugsweise besteht der Elektrodenträger aus Leiterplattenmaterial, das auf der Rückseite mit Leiterbahnen versehen ist und die elektrischen Bauelemente der Meßschaltung trägt. Das Massepotential der Meßschaltung 84 ist über eine Leitung (nicht dargestellt) zur Ringfläche 28 des Halterungsrings 10 geführt.

Das Ausführungsbeispiel nach der Figur 7 hat den Vorteil, daß es aufgrund der Aufhängung mittels eines Gummibandes 92 sehr reibungsarm bewegbar ist und keine Tiefenverstellung benötigt. Aufgrund der ebenen Elektrodenträgerfläche 96 hat es einen einfachen Aufbau und kann wirtschaftlich hergestellt werden.

Figur 8 zeigt eine schematische Draufsicht auf die Aufhängung mittels des Gummibandes 92 beim Ausführungsbeispiel nach Figur 7. Das Gummiband 92 ist sternförmig um die Haken 94 des Halterungsrings 10 und des Adaptergehäuses 90 geführt. Die Haken 94 sind im gleichmäßigen Abstand längs des Umfangs des Halterungsrings 10 bzw. des Adaptergehäuses 90 gegeneinander versetzt angeordnet. An Stelle des Gummibandes 92 kann auch eine Spirallängsfeder verwendet werden.

Es ist noch anzumerken, daß verschiedene Merkmale des Ausführungsbeispiels nach der Figur 7, wie beispielsweise die halbkreisförmigen Elektroden 98, die Art der Aufhängung mittels Gummibänder 92, das Dämpfungsmittel in Form einer Schaumgummieinlage 110 oder die elastische Schutzkappe 104 auch beim Ausführungsbeispiel nach der Figur 3 verwendet werden können.

## Patentansprüche

1. Vorrichtung zum Messen der Herzwandbewegungen einer Person, umfassend einen an die Brust anlegbaren Meßkopf (10, 12) mit einer Elektrodenanordnung (76, 78) und einer Meßschaltung (84) zur kapazitiven Messung der Hautbewegung gegenüber der Elektrodenanordnung (76, 78), dadurch **gekennzeichnet**, daß der Meßkopf einen Halterungsring (10) umfaßt, der eine zur Anlage am Körper (30, 32) bestimmte, zur Ringachse (26) senkrechte Ringfläche (28) und Mittel (22, 24) zur Befestigung des Meßkopfes (10, 12) am Körper hat, daß in dem Halterungsring (10) ein die Elektrodenanordnung (76, 78, 98) umfassender Adapter (12) in Richtung der Ringachse (26) sowie um zwei zu dieser und zueinander senkrechte Achsen federnd beweglich gelagert ist und daß der Adapter (12) eine zur Anlage am Körper bestimmte ringförmige Auflagefläche (42) und einen von dieser umschlossenen Elektrodenträger (56) mit einer mindestens eine Elektrode (76, 78, 98) tragenden Elektrodenträgerfläche (74, 96) hat, die senkrecht zur Ebene der Auflagefläche (42) einen Abstand hat und die mit einem Abstandselement (80, 100) versehen ist.

2. Vorrichtung nach Anspruch 1, dadurch **gekennzeichnet**, daß die Elektrodenträgerfläche (74) konkav ist und als Abstandselement in ihrem Zentrum eine Erhebung (80) hat, wobei die Elektrode (76, 78) im ringförmigen Abschnitt der Elektrodenträgerfläche (74) zwischen der Auflagefläche (42) und der Erhebung (80) angeordnet ist.

3. Vorrichtung nach Anspruch 1 oder 2, dadurch **gekennzeichnet**, daß der Elektrodenträger (56) relativ zur Auflagefläche (42) in Richtung der Ringachse (26) verstellbar ist.

4. Vorrichtung nach einem der Ansprüche 1 bis 3, dadurch **gekennzeichnet**, daß der Adapter (12) ein topfförmiges Gehäuse (34) hat, an dessen Öffnungsrand die Auflagefläche (42) ausgebildet ist und in dem der Elektrodenträger (56) zusammen mit der Meßschaltung (84) angeordnet ist.

5. Vorrichtung nach Anspruch 3 und 4, dadurch **gekennzeichnet**, daß der Elektrodenträger (56) durch Federmittel (68) in Richtung auf den Topfboden (50) vorgespannt und durch eine Stellvorrichtung (70, 72) von dem Gehäuseboden (50) weg bewegbar ist.

6. Vorrichtung nach Anspruch 5, dadurch **gekennzeichnet**, daß der Gehäuseboden (50) gegenüber der Gehäusewand (38) um die Ringachse (26) drehbar ist und an seiner Innenseite mindestens einen Nocken (72) trägt, der mit einer gegenüber einer achsnormalen Ebene geneigten Steuerfläche (70) an dem Elektrodenträger (56) zusammenwirkt.

7. Vorrichtung nach einem der Ansprüche 1 bis 6, dadurch **gekennzeichnet**, daß die ringförmige Auflagefläche (42) des Adapters (12) elektrisch leitend ist und Massepotential der Meßschaltung (84) führt.

8. Vorrichtung nach Anspruch 1, dadurch **gekennzeichnet**, daß die Elektrodenträgerfläche (96) eben ist und als Abstandselement eine Scheibe (100) vorgesehen ist.

9. Vorrichtung nach Anspruch 8, dadurch **gekennzeichnet**, daß die Elektrodenträgerfläche (96) kreisförmig ist und ein Ringelement (102) vorgegebener Höhe trägt, das vorzugsweise als Austauschteil ausgebildet ist.

10. Vorrichtung nach Anspruch 9, dadurch **gekennzeichnet**, daß die zum Körper zeigende Öffnung des Ringelements (102) mit einer elastischen Schutzkappe (104) verhüllt ist.

11. Vorrichtung nach Anspruch 10, dadurch **gekennzeichnet**, daß der zwischen der Schutzkappe (104), dem Ringelement (102) und der Scheibe (100) gebildete Raum mindestens eine Entlüftungsöffnung (106) zum Außenraum hat.

12. Vorrichtung nach einem der vorhergehenden Ansprüche, dadurch **gekennzeichnet**, daß der Adapter (12) im Halterungsring (10) durch Federmittel (48, 68, 92) federnd gelagert ist.

13. Vorrichtung nach Anspruch 12, dadurch **gekennzeichnet**, daß der Adapater (12) im Halterungsring (10) durch mindestens ein Gummiband (92) federnd gelagert ist, das abwechselnd am Adapter (12) und am Halterungsring (10) an mehreren Stellen gehalten ist.

14. Vorrichtung nach einem der vorhergehenden Ansprüche, dadurch **gekennzeichnet**, daß der Halterungsring (10) eine Gehäusekappe (108) trägt.

15. Vorrichtung nach Anspruch 14, dadurch **gekennzeichnet**, daß zwischen der von dem Körper abgewandten Seite des Adapters (12) und der Gehäusekappe (108) ein elastisches Dämpfungsmittel, vorzugsweise eine Schaumgummieinlage (110) angeordnet ist.

16. Vorrichtung nach einem der vorhergehenden Ansprüche, dadurch **gekennzeichnet**, daß die Meßschaltung (84) auf der vom Körper abgewandten Seite des aus Leiterplattenmaterial bestehenden Elektrodenträgers (56) angeordnet ist.

17. Vorrichtung nach einem der vorhergehenden Ansprüche, dadurch **gekennzeichnet**, daß die am Körper aufliegende Ringfläche (28) elektrisch leitend ist und Massepotential führt.

18. Vorrichtung nach einem der vorhergehenden Ansprüche, dadurch **gekennzeichnet**, daß die Elektrode (76, 78, 98)) ringförmig ausgebildet ist und gegebenenfalls die Erhebung (80) konzentrisch umgibt.

19. Vorrichtung nach einem der vorhergehenden Ansprüche, dadurch **gekennzeichnet**, daß zwei Elektroden (76, 78, 98) auf der Trägerfläche (74) angeordnet sind, die jeweils einem Oszillator der Meßschaltung (84) zugeordnet sind, wobei die Frequenzänderung der Oszillatoren bei Änderung der Kapazität des von der jeweiligen Elektrode (76, 78, 98) und der Haut gebildeten Kondensators ausgewertet wird.

20. Vorrichtung nach einem der Ansprüche 1 bis 18, dadurch **gekennzeichnet**, daß zwei Elektroden (76, 78, 98) zueinander auf der Trägerfläche (74) angeordnet sind, die an einen Oszillator der Meßschaltung (84) derart angeschlossen sind, daß jede Einzelkapazität die Dauer einer Halbwelle des Ausgangssignales bestimmt.

21. Vorrichtung nach Anspruch 19 oder 20, daß die Elektroden (76, 78) ringförmig konzentrisch zueinander auf der Trägerfläche (74) angeordnet sind.

22. Vorrichtung nach Anspruch 19 oder 20, dadurch **gekennzeichnet**, daß die Elektroden (98) halbkreisförmig sind.

23. Vorrichtung nach einem der Ansprüche 1 bis 22, dadurch **gekennzeichnet**, daß der Halterungsring (10) mit Befestigungsgurten (22, 24) verbunden ist.

24. Vorrichtung nach einem der Ansprüche 14 bis 22, dadurch **gekennzeichnet**, daß die Gehäusekappe (108) abgerundet und zwischen der Haut und einem elastischen Gurt gehalten ist.

## Claims

1. A device for monitoring the movements of cardiac wall segments of a person, comprising a measuring head (10, 12) placeable onto the breast and having an electrode arrangement (76, 78) and a measuring circuit (84) for capacitively measuring the skin movement relative to said electrode arrangement (76, 78), **characterized** in that said measuring head comprises a holding ring (10) having an annular surface (28) perpendicular to the ring axis (26) adapted for engagement with the body (30, 32), and means (22, 24) for fastening said measuring head (10, 12) to the body, in that an adapter (12) comprising said electrode arrangement (76, 78, 98) is resiliently movably arranged in said holding ring (10) in the direction of said ring axis (26) as well as about two axes perpendicular to said ring axis (26) and to one another, and that said adapter (12) has an annular contact surface (42) adapted for engagement with the body and an electrode carrier (56) surrounded by said annular contact surface (42) and having an electrode carrying surface (74, 96) carrying at least one electrode (76, 78, 98), said electrode carrying surface (74, 96) being spaced perpendicularly from the plane of said contact surface (42) and being provided with a spacing member (80, 100).

2. A device according to claim 1, **characterized** in that said electrode carrying surface (74) is concave and has an elevation (80) in its center, said elevation (80) serving as a spacing member, wherein said electrode (76, 78) is arranged in the annular portion of said electrode carrying surface (74) between said contact surface (42) and said elevation (80).

3. A device according to claim 1 or 2, **characterized** in that said electrode carrier (56) is adjustable relative to said contact surface (42) in the direction of said ring axis (26).

4. A device according to one of claims 1 to 3, **characterized** in that said adapter (12) has a pot-shaped housing (34) on the opening edge of which said contact surface (42) is formed, and in which said electrode carrier (56) is arranged together with said measuring circuit (84).

5. A device according to claims 3 and 4, **characterized** in that said electrode carrier (56) is biased in the direction towards the bottom (50) of said pot-shaped housing by spring means (68), and is movable away from the bottom (50) of said housing by an adjustment device (70, 72).

6. A device according to claim 5, **characterized** in that the bottom (50) of said housing is rotatable relative to the housing wall (38) about said ring axis (26) and on its inner side carries at least one cam (72) which cooperates with a control surface (70) on said electrode carrier (56), said control surface (70) being inclined with respect to an axis normal plane.

7. A device according to one of claims 1 to 6, **characterized** in that said annular contact surface (42) of said adapter (12) is electrically conductive and carries the ground potential of said measuring circuit (84).

8. A device according to claim 1, **characterized** in that said electrode carrying surface (96) is flat and that a disk (100) is provided to serve as a spacing member.

9. A device according to claim 8, **characterized** in that said electrode carrying surface (96) is circular and carries a ring member (102) of a predetermined height which preferably is designed as an exchangeable part.

10. A device according to claim 9, **characterized** in that the opening of said ring member (102) directed towards the body is covered with an elastic protective cap (104).

11. A device according to claim 10, **characterized** in that the space formed between said protective cap (104), said ring member (102) and said disk (100) has at least one air vent opening (106) to the ambient space.

12. A device according to one of the preceding claims, **characterized** in that said adapter (12) is resiliently arranged in said holding ring (10) by spring means (48, 68, 92).

13. A device according to claim 12, **characterized** in that said adapter (12) is resiliently arranged in said holding ring (10) by at least one rubber band (92) which is alternatingly held at several places on said adapter (12) and on said holding ring (10).

14. A device according to one of the preceding claims, **characterized** in that said holding ring (10) carries a housing cap (108).

15. A device according to claim 14, **characterized** in that a resilient dampening means, preferably a foam rubber insert (110) is arranged between the side of said adapter (12) facing away from the body and the housing cap (108).

16. A device according to one of the preceding claims, **characterized** in that said measuring circuit (84) is arranged on the side of said electrode carrier (56) facing away from the body, said electrode carrier (56) consisting of circuit board material.

17. A device according to one of the preceding claims, **characterized** in that said ring surface (28) engageable with the body is electrically conductive and carries ground potential.

18. A device according to one of the preceding claims, **characterized** in that said electrode (76, 78, 98) is ring-shaped and concentrically surrounds the elevation (80) if said elevation (80) is present.

19. A device according to one of the preceding claims, **characterized** in that two electrodes (76, 78, 98) are arranged on said carrier surface (74), each of said electrodes being assigned to an oscillator of said measuring circuit (84), wherein the frequency change of said oscillators upon a change of capacity of the capacitor formed by each of the electrodes (76, 78, 98) and the skin is evaluated.

20. A device according to one of claims 1 to 18, **characterized** in that two electrodes (76, 78, 98) are arranged relative to one another on said carrier surface, which electrodes are connected to an oscillator of said measuring circuit (84) in such a way that each individual capacity determines the duration of one half wave of the output signal.

21. A device according to claim 19 or 20, **characterized** in that said electrodes (76, 78) are arranged in an annular shape concentrically to one another on said carrier surface (74).

22. A device according to claim 19 or 20, **characterized** in that said electrodes (98) are semicircular.

23. A device according to one of claims 1 to 22, **characterized** in that said holding ring (10) is connected to fastening straps (22, 24).

24. A device according to one of claims 14 to 22, **characterized** in that said housing cap (108) has a rounded shape and is held between the skin and an elastic band.

## Revendications

1. Dispositif de mesure des mouvements de la paroi cardiaque d'une personne, comportant une tête de mesure (10, 12) pouvant être placée sur la poitrine avec un ensemble d'électrodes (76, 78) et un circuit de mesure (84) pour la mesure capacitive du déplacement de la peau par rapport à l'ensemble des électrodes (76, 78), caractérisé en ce que la tête de mesure comporte un anneau de fixation (10), qui possède une surface annulaire (28), destinée à s'appliquer sur le corps (30, 32), perpendiculaire à l'axe (26) de l'anneau ainsi que des moyens (22, 24) de fixation de la tête de mesure (10, 12) sur le corps, en ce que dans l'anneau de fixation (10) il est prévu un adaptateur (12) comprenant l'ensemble d'électrodes (76, 78, 98), mobile élastiquement en direction de l'axe (26) de l'anneau ainsi qu'autour de deux axes perpendiculaires à celui-ci et perpendiculaires entre eux, et en ce que l'adaptateur (12) présente une surface d'application (42) annulaire, destinée à s'appliquer contre le corps, ainsi qu'un porte-électrodes (56), entouré par cette surface d'application, avec une surface porteélectrodes (74, 96), portant au moins une électrode (76, 78, 98), laquelle surface est écartée perpendiculairement au plan de la surface d'application (42) et est pourvue d'un élément d'écartement (80, 100).

2. Dispositif selon la revendication 1, caractérisé en ce que la surface porte-électrodes (74) est concave et possède en son centre, comme élément d'écartement, un relief (80), l'électrode (76, 78) étant placée dans la partie annulaire de la surface porte-électrodes (74), entre la surface d'application (42) et le relief (80).

3. Dispositif selon les revendications 1 ou 2, caractérisé en ce que le porte-électrodes (56) est déplaçable par rapport à la surface d'application (42), en direction de l'axe (26) de l'anneau.

4. Dispositif selon l'une des revendications 1 à 3, caractérisé en ce que l'adaptateur (12) possède un boîtier (34) en forme de pot, sur le bord d'ouverture duquel est formée la surface d'application (42) et dans lequel le porte-électrodes (56) est placé avec le circuit de mesure (84).

5. Dispositif selon les revendications 3 et 4, caractérisé en ce que le porte-électrodes (56) est précontraint par des moyens à ressort (68) en direction du fond (50) du pot et peut être éloigné du fond (50) du boîtier par un dispositif de réglage (70, 72).

6. Dispositif selon la revendication 5, caractérisé en ce que le fond (50) du boîtier peut tourner par rapport à la paroi (38) du boîtier autour de l'axe (26) de l'anneau et porte, sur sa face intérieure, au moins une came (72), qui coopère avec une surface de commande (70) du porte-électrodes (56), inclinée par rapport à un plan normal à l'axe.

7. Dispositif selon l'une des revendications 1 à 6, caractérisé en ce que la surface d'application (42) annulaire de l'adaptateur (12) est électriquement conductrice et conduit le potentiel de masse du circuit de mesure (84).

8. Dispositif selon la revendication 1, caractérisé en ce que la surface porte-électrodes (96) est plane et une rondelle (100) est prévue comme élément d'écartement.

9. Dispositif selon la revendication 8, caractérisé en ce que la surface porte-électrodes (96) est circulaire et porte un élément annulaire (102) de hauteur donnée, qui est de préférence conçu comme une pièce interchangeable.

10. Dispositif selon la revendication 9, caractérisé en ce que l'ouverture dirigée vers le corps de l'élément annulaire (102) est enveloppée dans un capuchon de protection (104) élastique.

11. Dispositif selon la revendication 10, caractérisé en ce que l'espace formé entre le capuchon de protection (104), l'élément annulaire (102) et la rondelle (100) possède au moins une ouverture d'aération (106) dirigée vers le volume extérieur.

12. Dispositif selon l'une des revendications précédentes, caractérisé en ce que l'adaptateur (12) est monté dans l'anneau de fixation (10), de manière élastique, grâce à des moyens à ressort (48, 68, 92).

13. Dispositif selon la revendication 12, caractérisé en ce que l'adaptateur (12) est monté dans l'anneau de fixation (10), de manière élastique, grâce à au moins une bande en caoutchouc (92), qui est fixée alternativement sur l'adaptateur (12) et sur l'anneau de fixation (10), en plusieurs points.

14. Dispositif selon l'une des revendications précédentes, caractérisé en ce que l'anneau de fixation (10) porte un capuchon de boîtier (108).

15. Dispositif selon la revendication 14, caractérisé en ce qu'il est prévu entre le côté de l'adaptateur (12), tourné à l'opposé du corps, et le capuchon de boîtier (108), un moyen d'amortissement élastique, de préférence une garniture en caoutchouc mousse (110).

16. Dispositif selon l'une des revendications précédentes, caractérisé en ce que le circuit de mesure (84) est prévu sur le côté, tourné à l'opposé du corps, du porte-électrodes (56), réalisé dans un matériau pour plaque de circuits imprimés.

17. Dispositif selon l'une des revendications précédentes, caractérisé en ce que la surface annulaire (28), reposant sur le corps, est électriquement conductrice et conduit le potentiel de masse.

18. Dispositif selon l'une des revendications précédentes, caractérisé en ce que l'électrode (76, 78, 98) est annulaire et entoure concentriquement éventuellement le relief (80).

19. Dispositif selon l'une des revendications précédentes, caractérisé en ce que deux électrodes (76, 78, 98), à chacune desquelles est associé un oscillateur du circuit de mesure (84), sont placées sur la surface de support (74), la variation de la fréquence des oscillateurs, en cas de variation de la capacité du condensateur formé par l'électrode respective (76, 78, 98) et la peau, étant exploitée.

20. Dispositif selon l'une des revendications 1 à 18, caractérisé en ce que sur la surface de support (74) sont placées l'une par rapport à l'autre deux électrodes (76, 78, 98), qui sont raccordées à un oscillateur du circuit de mesure (84), de manière que chaque capacité individuelle détermine la durée d'une demi-onde du signal de sortie.

21. Dispositif selon les revendications 19 ou 20, caractérisé en ce que les électrodes (76, 78) sont disposées en anneaux concentriques les unes par rapport aux autres sur la surface de support (74).

22. Dispositif selon les revendications 19 ou 20, caractérisé en ce que les électrodes (98) sont en forme de demi-cercles.

23. Dispositif selon l'une des revendications 1 à 22, caractérise en ce que l'anneau de fixation (10) est relié à des sangles de fixation (22, 24).

24. Dispositif selon l'une des revendications 14 à 22, caractérisé en ce que le capuchon de boîtier (108) est arrondi et est maintenu entre la peau et une sangle élastique.
